# EUROPEAN PATENT SPECIFICATION

(11) **EP 3 075 377 B1**
(45) Date of publication and mention of the grant of the patent: **08.12.2021**
(21) Application number: 16182457.8
(22) Date of filing: 02.08.2016
(51) Int. Cl.: A61K 9/16, A61K 36/185, A61K 36/28, A61P 13/02

(54) **ORAL COMPOSITIONS COMPRISING POWDERED PLANT EXTRACTS**
ORALE ZUSAMMENSETZUNGEN UMFASSEND PULVERFÖRMIGE PFLANZENEXTRAKTE
COMPOSITIONS ORALES COMPRENANT DES EXTRAITS DE PLANTES EN POUDRE

(30) Priority: 18.03.2016 EP 16161027
(43) Date of publication of application: 05.10.2016
(73) Proprietor: HERMES PHARMA GmbH, 82049 Pullach (DE)
(72) Inventor: HÖBART, Hans, 82049 Pullach (DE); GARSUCH, Verena, 82049 Pullach (DE); LANG, Claudia, 82335 Berg (DE)
(74) Representative: Pharma Patents International AG

(56) References cited:
- CN-A- 1 899 341
- KR-A- 20120 053 305
- RU-C1- 2 197 980
- UA-C2- 37 829
- Anonymous: "Methylcellulose" In: "Handbook of Pharmaceutical Excipients", 1 January 2009 (2009-01-01), Pharmaceutical Press, XP055598907, ISBN: 978-0-85369-792-3 pages 438-441,

## Description

### FIELD

The present invention relates to compositions comprising powdered plant extracts, e.g. extracts of birch leaves or solidago leaves; in particular, dry flowable compositions intended for dissolution in water in order to provide a drinking solution of the plant extract. The present invention further relates to compositions used in the treatment and/or prevention of urinary tract infections.

### BACKGROUND

In the field of bacterial urinary tract infections (UTIs), the use of plant extracts is still very common. Known plants and/or plant components exhibiting for instance a diuretic effect include bearberry leaves (or meal berry), birch leaves, solidago leaves (also called goldenrod), orthosiphon leaves, dandelion, horsetail herbs, parsley, restharrow roots, juniper berries, black currant leaves, garden lovage and asparagus. Other plant extracts are assumed to prevent or limit the attachment of UTI-inducing bacteria to the bladder mucosa, the so-called urothelium; e.g. cranberry extract.

CN 1 899 341 A discloses granules for oral administration comprising a dried extract of American goldenrod herb, a carrier mixture of micronised silica gel, lactose, and starch, as well as polysorbate 80 as a surfactant. The granule composition is intended for cystitis treatment. One of the disadvantages of the suggested compositions of CN 1 899 341 A, however, is that the carrier mixture contains rather high amounts of the water-insoluble, or only partially water-soluble, substances silica gel and starch; the amount of the latter two being almost as high as the amount of the water-soluble lactose. This renders the granules unsuitable for the intended dissolution in water to provide a drinking solution since large amounts of insoluble components in a drinking solution are both unsightly and unpleasant to swallow.

Since it is generally recommended to increase the amount of consumed liquids during UTIs in order to remove as many bacteria as possible via the urine, drinking solutions present an advantageous vehicle for the plant extracts. As a further benefit, drinking solutions are easily swallowable by subjects of any age. Hence, some of the plant extracts are sold as alcoholic solutions; e.g. as concentrated drops, often to be taken with a bit of sugar (often sucrose) or diluted in water to mask their taste. However, this is not an appropriate approach for e.g. infants, kids or any subject which does not want to consume alcohol, either due to alcoholism, religious concerns and/or risk of affecting comedication.

One approach was to provide powdered plant extracts, either milled or obtained by spray-drying or freeze-drying liquid extracts. A common issue with at least some of these powdered plant extracts, though, is that they tend to self-agglomerate and form lumps upon dispersion into water, leading to reduced dissolution rates and in some cases sediments. In some but not all cases, this problem may be overcome to some degree by vigorous stirring; yet often such stirring then causes the mixture to foam, which is unsightly and may affect dosing accuracy.

It is thus an object of the present invention to provide compositions for oral delivery of powdered plant extracts which can be stored in dry state and easily dispersed and dissolved in water prior to consumption without the formation of foam, agglomerates and/or sediments. A further object is to provide compositions for use in a method for the treatment and/or prevention of urinary tract infections such as cystitis, wherein said compositions, for instance comprise birch leaf extract and/or solidago leaf extract.

Further objects will become apparent on the basis of the following description including the examples, and the patent claims.

### SUMMARY OF THE INVENTION

In a first aspect, the invention provides a dry flowable composition comprising
granules, said granules comprising (a) a powdered plant extract, (b) a carrier mixture comprising a water-soluble substance selected from a sugar and/or sugar alcohol and a separating agent at a weight ratio of 1 or higher, and (c) a surfactant;
wherein the sugar and/or sugar alcohol is selected from glucose, fructose, mannose, sucrose, maltose, lactose, maltitol, mannitol, sorbitol, xylitol, erythritol, isomalt, or mixtures thereof; and/or
the separating agent is selected from calcium carbonate, calcium phosphate, magnesium oxide, magnesium carbonate, fumaric acid, or mixtures thereof,
and wherein the granules comprise from 10 to 70 wt.-% powdered plant extract; from 20 to 80 wt.-% sugar and/or sugar alcohol; from 3 to 20 wt.-% separating agent; and from 0.01 to 3 wt.-% surfactant.

The powdered plant extract may for instance be a birch leaf extract and/or a solidago leaf extract, the birch leaf extract optionally comprising at least 2 % flavonoids.

In a more specific embodiment, the granules comprise from about 30 to about 50 wt.-% powdered plant extract; from about 40 to about 60 wt.-% sugar and/or sugar alcohol; from about 5 to about 10 wt.-% separating agent; and/or from about 0.05 to about 1 wt.-% surfactant.

The weight ratio of the sugar and/or sugar alcohol and the separating agent in the carrier mixture of the granules ranges from 4 :1 to 11 : 1, preferably from about 5.5 : 1 to about 8.5 : 1.

The granules may for instance comprise sucrose, calcium carbonate as the separating agent and polysorbate as the surfactant (e.g. polysorbate 20).

The dry flowable compositions may further comprise mannose (preferably D-mannose), optionally at an amount of about 10 to about 70 wt.-%; and/or one or more vitamins, e.g. vitamin A and/or vitamin D3. Furthermore, the compositions may comprise one or more, preferably extragranular, excipients selected from edible acids (e.g. citric acid, tartaric acid, succinic acid, malic acid or lactic acid, or combinations thereof); pH-modifying agents such as citrates; natural and/or artificial flavours; anti-foaming agents such as medium chain triglycerides (MCTs) with 6 to 12 carbon atoms or simethicone; and/or sweeteners (e.g. aspartame, sucralose, cyclamate, neotame, neohesperidin, acesulfame potassium, saccharin, advantame, thaumatin, stevia, stevioside, and/or mixtures thereof).

A typical composition according to the invention may comprise about 30 to 60 % mannose; about 5 to 20 % powdered birch leaf extract and/or powdered solidago leaf extract; about 10 to 30 % sucrose; about 0.5 to 3 % calcium carbonate; about 5 to 25 % citric acid; about 3 to 15 % trimagnesium dicitrate; about 3 to 15 % monosodium citrate; and about 0.001 to 2 % surfactant.

In a further aspect, the invention provides a process for the preparation of the dry flowable composition according to the invention, comprising the steps of
a) mixing the sugar and/or sugar alcohol and the separating agent,
b) adding a solution comprising an alcohol selected from ethanol or isopropanol, and optionally a surfactant, to the mixture of step a to form a carrier mixture,
c) adding the powdered plant extract to the carrier mixture of step b, and
d) drying the product of step c, and
e) adding to the product of step d mannose and/or vitamin(s) and/or at least one extragranular excipient selected from edible acids (e.g. citric acid, tartaric acid, succinic acid, malic acid or lactic acid, or combinations thereof); pH-modifying agents such as citrates; natural and/or artificial flavours; anti-foaming agents such as medium chain triglycerides (MCTs) with 6 to 12 carbon atoms or simethicone; and/or sweeteners (e.g. aspartame, sucralose, cyclamate, neotame, neohesperidin, acesulfame potassium, saccharin, advantame, thaumatin, stevia, stevioside, and/or mixtures thereof).

In a further aspect, the invention provides a single dose unit or package comprising the dry flowable composition according to the invention. Typically, the amount of the total composition is about 2000 to 6000 mg, the amount of granules in said composition is about 800 to 1200 mg, and/or the amount of flavonoids is about 5 to 20 mg.

In a yet further aspect, the invention provides an oral liquid composition obtained from dissolving or dispersing the dry flowable composition of the invention, or the single dose unit thereof in water or an aqueous ingestible liquid.

In a further aspect, the invention provides the use of the dry flowable composition as well as single dose units or oral liquid composition thereof in the treatment and/or prevention of urinary tract infections, such as cystitis.

### Definitions

The following terms or expressions as used herein should normally be interpreted as outlined in this section, unless defined otherwise by the description or unless the specific context indicates or requires otherwise:
All technical terms as used herein shall be understood to have the same meaning as is commonly understood by a person skilled in the relevant technical field.

The words 'comprise', 'comprises' and 'comprising' and similar expressions are to be construed in an open and inclusive sense, as 'including, but not limited to' in this description and in the claims.

The terms "a" or "an" as used herein means "at least one" or as "one or more" unless specified otherwise; i.e. it does not exclude pluralities. In other words, all references to singular characteristics or limitations of the present disclosure shall include the corresponding plural characteristic or limitation, and vice versa.

All percentages, parts and ratios as used herein, are by weight of the respective total weight, unless otherwise specified. For instance "%" is to be understood as "wt.-%" unless otherwise specified.

As used herein terms like "about", "approximately" or "ca." shall compensate for any variability allowed for in the pharmaceutical and/or nutraceutical industry and inherent in their respective products, such as differences in content due to manufacturing variations and/or time-induced product degradation.

The expressions, 'one embodiment', 'an embodiment', 'a specific embodiment' and the like mean that a particular feature, property or characteristic, or a particular group or combination of features, properties or characteristics, as referred to in combination with the respective expression, is present in at least one of the embodiments of the invention. These expressions, occurring in various places throughout this description, do not necessarily refer to the same embodiment. Moreover, the particular features, properties or characteristics may be combined in any suitable manner in one or more embodiments

The term "sugar" refers to sweet, short-chain, soluble carbohydrates from various sources. The term includes monosaccharides (such as glucose, fructose or mannose), and disaccharides (such as sucrose, maltose, lactose, lactulose, maltulose, trehalose or palatinose).

The term "sugar alcohol" refers to organic compounds of the general formula: HOCH₂(CHOH)ₙCH₂OH. They are typically derived from sugar and, unlike sugars, usually linear molecules. They are also known as polyhydric alcohols, polyalcohols, alditols or glycitols. Common examples of sugar alcohols include mannitol, maltitol, sorbitol, xylitol, erythritol, isomalt, lactitol or inositol.

A "separating agent" as used herein is a solid, fine powdered substance, typically below 10 µm particle size, which is practically insoluble, very slightly soluble or at most slightly soluble in water at room temperature; and which is capable of covering, or coating the surface of other solid particles, typically particles with a larger particle size than the separating agent. Any solubility provisions such as "slightly soluble" as used herein shall be understood as aqueous solubilities and ranked according to pharmacopoeial standards (e.g. European Pharmacopeia) unless specified otherwise.

Any particle size provisions such as <20 µm or <10 µm shall be understood as sieve diameters; i.e. particles of a sieve diameter <20 µm would normally pass through a sieve having an aperture, or opening size, of 20 µm. Within a formulation comprising a plurality of particles, these particle sizes should be interpreted to characterise the preferred mass median sieve diameters of particles (so-called D50 value), unless where provided otherwise. The D90-value refers to the particle size of the sieve which 90 % of the particle would pass. It is to be understood that commonly the particle size values of D50 and/or D90 are calculated from the measured particle size distribution. The latter may be measured using laser diffraction or other dynamic imaging techniques using e.g. pulsed LED light.

### DETAILED DESCRIPTION OF THE INVENTION

In a first aspect, the invention provides a dry flowable composition comprising
granules, said granules comprising (a) a powdered plant extract, (b) a carrier mixture comprising a water-soluble substance selected from a sugar and/or sugar alcohol and a separating agent at a weight ratio of 1 : 1 or higher, and (c) a surfactant;
wherein the sugar and/or sugar alcohol is selected from glucose, fructose, mannose, sucrose, maltose, lactose, maltitol, mannitol, sorbitol, xylitol, erythritol, isomalt, or mixtures thereof; and/or
the separating agent is selected from calcium carbonate, calcium phosphate, magnesium oxide, magnesium carbonate, fumaric acid, or mixtures thereof,
and wherein the granules comprise from 10 to 70 wt.-% powdered plant extract; from 20 to 80 wt.-% sugar and/or sugar alcohol; from 3 to 20 wt.-% separating agent; and from 0.01 to 3 wt.-% surfactant.

In one specific embodiment of the invention, the powdered plant extract is a birch leaf extract and/or a solidago leaf extract. In a more specific embodiment, the powdered plant extract is a birch leaf extract comprising at least 2 % flavonoids. Earlier attempts to formulate such powdered birch leaf extracts and/or solidago leaf extracts into drinking solutions without the inventive granules suffered from noticeable, unsightly foam formation and poor dissolution with residues/sediments remaining at the bottom of the drinking vessel. The foam formation is presumed to be associated - at least in parts - with the saponin content in birch leaf extracts and solidago leaf extracts; though the 'foaming problem' is by no means limited to saponin-containing plant extracts. Similar problems are known from other powdered plant extracts such as sage (clary, salvia), ginseng, horse chestnut or gynostemma (Jiaugulan), harpagophytum, echinacea or urtica.

This problem was successfully overcome by the granules of the invention. The inventors surprisingly found that granules based on the carrier mixture provide improved dissolution properties to powdered plant extracts, characterised by limited foam formation during stirring and complete and rapid dissolution in cold water, typically within less than 30-40 seconds after short stirring (e.g. about 10 seconds).

The mechanism for this improved dissolution performance is not yet understood in detail. One aspect is that the combination of a powdered plant extract with a water-soluble substance selected from a sugar and/or sugar alcohol and a separating agent helps to prevent the agglomeration of the powdered plant extract by coating the surface of the water-soluble substance with it, thereby separating and/or dispersing the powdered plant extract. It is further believed that upon dissolution of the granules, the main carrier mixture component - namely the sugar and/or sugar alcohol - allows for rapid dissolution of the granules, while the separating agent ensures that the powdered plant extract particles do not 're-agglomerate' and hence also dissolve quicker as they would without the carrier mixture.

As defined above, the separating agent is a finely powdered, poorly soluble material (at most slightly soluble), with a particle size that enables the agent to cover, or coat, surfaces of larger sized particles evenly. Due to its larger surface area per weight, the amount of separating agent needed is smaller than the amount of the water-soluble main carrier mixture component, the sugar and/or sugar alcohol; hence, the ratio of sugar and/or sugar alcohol and the separating agent in the carrier mixture of the granules ranges from 4 : 1 to 11 : 1. In one embodiment, the weight ratio is from about 5.5 : 1 to about 8.5 : 1; for instance 6 : 1, 6.5 : 1, 7 : 1, 7.5 : 1 or 8 : 1. In one of the preferred embodiments, the ratio is about 7 : 1.

The sugar and/or sugar alcohol is selected from glucose, fructose, mannose, sucrose, maltose, lactose, maltitol, mannitol, sorbitol, xylitol, erythritol, isomalt, or mixtures thereof; and/or the separating agent is selected from calcium carbonate, calcium phosphate, magnesium oxide, magnesium carbonate, fumaric acid, or mixtures thereof. Any combination is possible as long as the selected separating agent is capable of adhering to the surface of the sugar and/or sugar alcohol.

In one embodiment, the sugar and/or sugar alcohol is sucrose. In one embodiment, the separating agent is calcium carbonate. In a specific embodiment, the sugar and/or sugar alcohol is sucrose, the separating agent is calcium carbonate, and/or the surfactant is a polysorbate. Optionally, the polysorbate is polysorbate 20 (also known commercially as Tween^{®} 20).

In one of the preferred embodiments, the sugar and/or sugar alcohol is sucrose, the separating agent is calcium carbonate and the surfactant is polysorbate 20. Exemplary formulations using this carrier mixture are described in the examples. In this embodiment, the sucrose is preferably used in particle sizes as commonly found in caster sugar; i.e. between about 100 and 1000 µm, or between about 200 and 800 µm; and the calcium carbonate in particle sizes below 20 µm, or below 10 µm, or with a D90-value below 10 µm, such as to allow good adherence of the calcium carbonate to the sucrose crystals' surfaces.

The granules in the dry flowable composition comprise from about 10 to about 70 % powdered plant extract; from about 20 to about 80 % sugar and/or sugar alcohol; from about 3 to about 20 % separating agent; and/or from about 0.01 to about 3 % surfactant. In a specific embodiment, the granules comprise about 30 to 50 % powdered plant extract; about 40 to 60 % sugar and/or sugar alcohol; about 5 to 10 % separating agent; and/or about 0.05 to 1 % surfactant. In another specific embodiment, the granules comprise about 35 to 45 % powdered plant extract; about 45 to 55 % sugar and/or sugar alcohol; about 6 to 9 % separating agent; and/or about 0.05 to 0.2 % surfactant. In one of the preferred embodiments, the granules comprise about 42 % powdered plant extract (e.g. birch leaf extract and/or solidago leaf extract), about 50 % sugar and/or sugar alcohol (e.g. sucrose); about 7 % separating agent (e.g. calcium carbonate) and about 0.1 % surfactant (e.g. polysorbate 20).

In one embodiment, the dry flowable composition further comprises mannose, optionally at an amount of about 10 to about 70 %. In cases where the composition is intended for use in the treatment and/or prevention of urinary tract infections (UTIs) such as cystitis, D-mannose is preferred, since it prevents or limits the attachment of UTI-inducing bacteria to the bladder mucosa, the so-called urothelium.

In addition or alternatively, the composition further comprises a vitamin. Optionally, the vitamin is selected from vitamin A (retinol), vitamin B5 (pantothenic acid), vitamin C (ascorbic acid), vitamin D3 (cholecalciferol) and vitamin E (tocopherol). These vitamins provide mucosal protection, as is preferred e.g. in the treatment and/or prevention of urinary tract infections (UTIs) such as cystitis and other infections involving mucosal tissue. In one specific embodiment, the vitamin is vitamin A, vitamin D3 or a combination thereof.

In one embodiment, the composition further comprises one or more preferably extragranular excipients selected from edible acids, pH-modifying agents, sweeteners, natural and/or artificial flavours, and/or anti-foaming agents.

Optionally, the edible acid is selected from citric acid, tartaric acid, succinic acid, malic acid and lactic acid or combinations thereof. Further optionally, the pH-modifying agent is selected from citrates; for instance from trimagnesium dicitrate, monosodium citrate, disodium citrate. In one embodiment, citric acid is used. In one of the preferred embodiments, citric acid is used in combination with monosodium citrate and/or trimagnesium dicitrate.

Further optionally, the sweetener is selected from aspartame, sucralose, cyclamate, neotame, neohesperidin, acesulfame potassium, saccharin, advantame, thaumatin, stevia or stevioside, or mixtures thereof.

Optionally, the natural and/or artificial flavours are selected from dried fruit juice or herbal extracts and/or artificial flavours such as honey-, orange-, lemon-, cherry-, tropical fruit-, grapes-, berries-, banana-, contramarum-, or peach aroma, or mixtures thereof. Artificial flavours may be nature identical.

Further optionally, the anti-foaming agent is selected from medium chain triglycerides (MCTs) with 6 to 12 carbon atoms, such as medium chain triglycerides (MCTs) with 8 to 12 carbon atoms (e.g. Miglyol^{®}812), or simethicone.

In one of the preferred embodiments, the dry flowable composition comprises from about 30 to about 60 % mannose; from about 5 to about 20 % powdered birch leaf extract and/or powdered solidago leaf extract; from about 10 to about 30 % sucrose; from about 0.5 to about 3 % calcium carbonate; from about 5 to about 25 % citric acid; from about 3 to about 15 % trimagnesium dicitrate; from about 3 to about 15 % monosodium citrate; and from about 0.001 to about 2 % surfactant.

In this embodiment, the powdered birch leaf extract and/or the powdered solidago leaf extract, the calcium carbonate, the polysorbate and parts of the sucrose content is provided in the form of granules. Yet, further sucrose may be added extragranular as well in order to optimise the taste of the composition. Further optional excipients for this embodiment include sweeteners, flavours, vitamins and anti-foaming agents such as medium chain triglycerides (MCTs) with 6 to 12 carbon atoms (e.g. Miglyol^{®}812) or simethicone.

In one embodiment, the dry flowable composition comprises about 35 to 45 % mannose; about 5 to 15 % powdered birch leaf extract and/or powdered solidago leaf extract; about 15 to 20 % sucrose; about 1 to 2 % calcium carbonate; about 10 to 15 % citric acid; about 5 to 10 % trimagnesium dicitrate; about 5 to 10 % monosodium citrate; and about 0.01 to 0.1 % surfactant.

In a specific embodiment, the dry flowable composition comprises about 43 % mannose; about 10 % powdered birch leaf extract and/or powdered solidago leaf extract; about 17 % sucrose (in total); about 2 % calcium carbonate; about 12 % citric acid; about 8 % trimagnesium dicitrate; about 7 % monosodium citrate; and about 1 % in total of polysorbate 20, vitamin A, vitamin D3, sucralose, aspartame, and a C₈-C₁₂-MCT (e.g. Miglyol^{®}812). An exemplary formulation is described in the examples below.

While the invention is particularly useful for powdered plant extracts which contain saponins and/or exhibit foaming when stirred into aqueous liquids, it should be understood that the invention is not limited to only those extracts. It is irrelevant from which type of plant the extract is derived (e.g. herbs, bushes, trees), or from which part of the plant (e.g. roots, leaves, flower, fruits) or by which extraction method and/or extraction medium.

It should further be understood, that depending on the specific extract to be obtained, the extraction method and/or the extraction medium, some powdered plant extracts as commercially available may comprise additional substances such as excipients, i.e. substances which were not derived, or extracted from the plant as such, but which are added during the preparation process of the powdered plant extracts. For instance, the commercially available powdered solidago leaf extract as employed in Examples 1-3 further below comprises 10 % maltodextrin.

In a further aspect the invention provides a process for the preparation of the dry flowable composition described above, the process comprising the steps of
a) mixing the sugar and/or sugar alcohol and the separating agent,
b) adding a solution comprising an alcohol selected from ethanol or isopropanol, and optionally a surfactant, to the mixture of step a to form a carrier mixture,
c) adding the powdered plant extract to the carrier mixture of step b, and
d) drying the product of step c, and
e) adding to the product of step d mannose and/or vitamin(s) and/or at least one extragranular excipient selected from edible acids, optionally selected from citric acid, tartaric acid, succinic acid, malic acid and/or lactic acid;
   pH-modifying agents, optionally selected from citrates; sweeteners, optionally selected from aspartame, sucralose, cyclamate, neotame, neohesperidin, acesulfame potassium, saccharin, advantame, thaumatin, stevia, stevioside, and/or mixtures thereof;
   natural and/or artificial flavours; and/or anti-foaming agents, optionally selected from medium chain triglycerides (MCTs) with 6 to 12 carbon atoms or simethicone.

The process may be performed in any common granulation device suited to homogenously apply the particles of the separating agent and the powdered plant extract to the surfaces of the water-soluble main component of the carrier mixture (i.e. the sugar and/or sugar alcohol). For instance, a 'single-pot' granulation device may be used, optionally equipped with vacuum means such as the Topo-granulator.

The sugar and/or sugar alcohol and the separating agent should not dissolve in the alcoholic solution, or at most to a negligible degree. This helps to spread small amounts of solution more easily between the two powders without the risk for agglomeration and to thereby cover, or coat, the surface of the sugar and/or sugar alcohol crystals evenly with the fine powdered separating agent. Hence, the water content in the alcoholic solution should be kept below 10 %, or preferably below 7 %. In one embodiment, the pure alcohol is used. In one of the preferred embodiments ethanol is used as the alcohol, for instance ethanol 96 % (V/V).

Optionally, a surfactant, such as a polysorbate, may be dissolved in the alcoholic, or ethanolic, solution in order to improve the wetting of the powders.

Once a homogenous mixture is obtained, the powdered plant extract is added and dispersed in the carrier mixture. Since most powdered plant extracts are at least partially soluble in alcoholic solutions, the extract will 'stick' to the wetted carrier mixture particles while the separating agent prevents the auto-agglomeration, or self-agglomeration, of the plant extract particles during this process. Like this, an even layer of plant extract (partially in particulate state, partially dissolved) is formed on top of the carrier mixture particles, yielding the granules of the invention.

The thus obtained granules are subsequently dried; optionally using increased temperatures and/or vacuum. For instance, the granules may be dried at 50 °C under vacuum, optionally within the 'single-pot' granulator. During the drying step, the alcohol should evaporate completely, such as to leave no alcohol (other than e.g. sugar alcohols) in the final product.

Since the size of the sugar and/or sugar alcohol crystals typically exceeds the particle sizes of the separating agent and the powdered plant extract by at least a multitude of two, only a small size increase of the sugar and/or sugar alcohol particles is noticeable after granulation, such that the dried product is ready for further processing. Nonetheless a sieving step may be performed - if considered expedient - to remove any oversized particles after the drying and prior to processing the granules further.

Alternatively, to the process described above, the powdered plant extract may also be added directly to the sugar and/or sugar alcohol and the separating agent in step (a); i.e. prior to the addition of the alcoholic solution. Whether sufficient homogeneity of the granulation mixture can be obtained with this approach, depends on a number of factors, such as the solubility of the extract in the alcoholic solution and/or the extent of its auto-agglomeration tendencies. It should be noted that this alternative process does not deviate from the current invention, as long as the plant extract and the separating agent can be applied evenly and homogenously to the sugar and/or sugar alcohol particles.

The dried granules of step (c) may then be mixed with any further excipients of the composition; for instance with mannose and/or vitamins and/or excipient selected from edible acids, pH-modifying agents, sweeteners, natural and/or artificial flavours and/or anti-foaming agents. It is understood that any mixing device, mixing duration and any order of adding the further components is allowed as long as the final dry flowable composition is homogenous. The final composition is then ready for packaging.

In a yet further aspect the invention provides a single dose unit or package comprising the dry flowable composition described above. The single dose unit may be packaged in any suitable packaging material, such as foil-lined sachets or stickpacks, or small vials or bottles made from either glass or plastic materials. In one embodiment of the single dose unit or package, the amount of the total dry flowable composition is from about 2000 to about 6000 mg, and the amount of granules in the composition is from about 800 to about 1200 mg; and/or wherein the amount of flavonoids is from about 5 to about 20 mg. In a specific embodiment, the amount of the total dry flowable composition is about 4000 to 5000 mg (e.g. about 4500 mg or about 4600 mg), and the amount of granules in the composition is about 1000 to 1100 mg (e.g. about 1050 mg); and/or wherein the amount of flavonoids is from about 8 to about 15 mg (e.g. about 10 mg or about 15 mg).

If the single dose unit comprises mannose and/or vitamins in addition to the granules, their respective amounts should be chosen in such a way as to not exceed the recommended daily intake when taking into account the intended number of single dose units to be administered per day.

The dry flowable composition and/or the single dose unit or package thereof are intended for dissolution in water or another aqueous ingestible liquid in order to prepare a drinking solution. Hence, the invention further provides an oral liquid composition obtained from dissolving the dry flowable composition according to the invention or the single dose unit or package thereof in water or an aqueous ingestible liquid. Such drinking solutions are on one hand advantageous in that they can be easily swallowed by subjects of any age; they may even be administered by nasal or oral feeding tubes if needed. A further advantage of drinking solutions is that they increase the fluid uptake of the subject as is particularly desirable in the treatment and/or the prevention of urinary tract infections, such as cystitis.

Hence, in a final aspect, the invention provides the dry flowable composition as described above, or the single dose unit or package thereof, or the oral liquid composition obtained thereof for use in the treatment and/or prevention of urinary tract infections.

In one of the preferred embodiments, a composition is used comprising both, a birch leaf extract and D-mannose; or a solidago leaf extract and D-mannose; or alternatively a combination of birch leaf and solidago leaf extract together with D-mannose. Like this a synergistic effect is obtained:
D-mannose prevents or limits the attachment of UTI-inducing bacteria to the bladder mucosa, the so-called urothelium; and
the birch leaf extract and/or the solidago leaf extract provide(s) a diuretic effect which helps to 'flush out' said bacteria quickly. Furthermore, vitamins A and D3 were found to be advantageous to protect the urothelium.

### Examples

### Example 1 - Preparation of plant extract granules

In a laboratory scale experiment, granules according to the invention (1) are prepared by blending sucrose and calcium carbonate and granulating them with an ethanolic solution comprising 1 % polysorbate 20 (e.g. 0.2 g Polysorbate dissolved up to 20 mL in ethanol) to improve the surface wetting of the sucrose and calcium carbonate particles. To the thus obtained carrier mixture, either powdered birch leaf extract (here with a flavonoid content of about 2.3 %; granules 1-3) or powdered solidago leaf extract (granules 4-6), or an exemplary 50:50 blend of both extracts (granules 7) is added gradually and mixed until homogeneity.

The resulting granules are dried to constant weight at 50 °C under vacuum.

For comparison, similar granules were prepared omitting either the use of the calcium carbonate (2, 5) or the use of the sucrose (3, 6). The composition of all three granules is provided in Table 1 below.

**Table 1: Composition (amounts in gram) of granules 1-7**

| **Component** | **1** | **2** | **3** | **4** | **5** | **6** | **7** |
|---|---|---|---|---|---|---|---|
| Sucrose | 10.5 | 12.0 | - | 10.5 | 12.0 | --- | 10.5 |
| Birch leaf extract | 8.7 | 8.7 | 8.7 | --- | --- | --- | 4.35 |
| Solidago leaf extract | --- | --- | --- | 8.7 | 8.7 | 8.7 | 4.35 |
| Calcium carbonate | 1.5 | --- | 12.0 | 1.5 | --- | 12.0 | 1.5 |
| Polysorbate 20 | 0.02* | 0.02* | 0.02* | 0.02* | 0.02* | 0.02* | 0.02* |

| | | | | | | | |
|---|---|---|---|---|---|---|---|
| * 0.2 g Polysorbate dissolved up to 20 mL in ethanol; 2 mL added to sucrose/CaCO₃ | | | | | | | |

### Example 2 - Preparation of dry flowable composition

About 1 part of the plant extract granules obtained in Example 1 are combined with about 4 parts further excipients and blended until homogeneity to form a dry flowable composition according to the invention. The components of the composition and their respective contents in wt.-% are provided in Table 2 below.

A single dose unit of this composition comprises about 2 g D-mannose, about 10 mg flavonoids from birch leaf extract (granules 1-3), about 120 µg Vitamin A and about 200 I.E. Vitamin D3 (cholecalciferol). The single unit may be packaged in a sachet or stickpack; alternatively, it may be packaged in ampoules, vials or small bottles.

Upon use, the dry flowable composition is dissolved in water (e.g. about 200 mL) and stirred for about 10 seconds or until the composition is completely dissolved and ready for oral consumption.

**Table 2: Components of a dry flowable composition and their contents in wt.-%**

| **Component** | **wt.-%** |
|---|---|
| D-Mannose | 43.48 |
| Birch leaf extract (comprising 2.3 % flavonoids), and/or Solidago leaf extract | 9.46 |
| Vitamin A-palmitate 250.000 IE/g | 0.05 |
| Cholecalciferol 100.000 SD/S | 0.05 |
| Sucrose | 17.05 |
| Citric acid (anhydrous) | 11.96 |
| Trimagnesium-dicitrate (anhydrous) | 7.83 |
| Monosodium citrate | 7.39 |
| Calcium carbonate | 1.63 |
| Aspartame | 0.59 |
| Aroma | 0.33 |
| Sucralose | 0.15 |
| Polysorbate 20 | 0.02 |
| Medium chain triglycerides (C₈ to C₁₂) | 0.02 |

### Example 3 - Dissolution behaviour of plant extract granules

The plant extract granules obtained in Example 1 are mixed with further ingredients in analogy to Example 2 and the resulting dry flowable compositions 1-3 were dissolved at room temperature in about 200 mL water and stirred for about 10 seconds. About 30 seconds after stirring the obtained drinking compositions are inspected visually.

The results are provided in Table 3 below.

**Table 3: Results of visual inspection of dissolution behaviour of compositions 1-3**

| **Granule** | **Observations** |
|---|---|
| **1** | Composition completely dissolved; very minor foam formation |
| **2** | Composition not completely dissolved; Brown sediment of plant extract remains even after 120 s |
| **3** | No visual plant extract sediments; but lots of persisting foam Taste and pH not optimal for repeated oral consumption |
| **4** | Composition completely dissolved; very minor foam formation |
| **5** | Composition not completely dissolved; Brown sediment of plant extract remains even after 120 s |
| **6** | No visual plant extract sediments; but lots of persisting foam Taste and pH not optimal for repeated oral consumption |
| **7** | Composition completely dissolved; very minor foam formation |

As can be seen from these results, only the combination of sucrose with the separating agent calcium carbonate provides the desired dissolution performance with compositions 1, 4 and 7 dissolving completely with only very minor foam formation. Without the use of the separating agent, the plant extract particles do not dissolve properly and form a sediment instead. This shows that the inventive effect does not reside in simply dispersing plant extract particles in sucrose particles to counter-act their agglomeration tendencies. On the other hand, without the use of the water-soluble sucrose (and more calcium carbonate instead), lots of persisting foam is formed upon stirring.

## Claims

1. A dry flowable composition comprising granules, said granules comprising
(a) a powdered plant extract,
(b) a carrier mixture comprising a water-soluble substance selected from a sugar and/or sugar alcohol and a separating agent at a weight ratio from 4 : 1 to 11 : 1, and
(c) a surfactant;
wherein the sugar and/or sugar alcohol is selected from glucose, fructose, mannose, sucrose, maltose, lactose, maltitol, mannitol, sorbitol, xylitol, erythritol, isomalt, or mixtures thereof; and/or
the separating agent is selected from calcium carbonate, calcium phosphate, magnesium oxide, magnesium carbonate, fumaric acid, or mixtures thereof, and wherein the granules comprise
from 10 to 70 wt.-% powdered plant extract;
from 20 to 80 wt.-% sugar and/or sugar alcohol;
from 3 to 20 wt.-% separating agent; and
from 0.01 to 3 wt.-% surfactant.

2. The composition of claim 1, wherein the powdered plant extract is birch leaf and/or a solidago leaf extract, the birch leaf extract optionally comprising at least 2 % flavonoids.

3. The composition of any preceding claim, wherein the weight ratio of the sugar and/or sugar alcohol and the separating agent in the carrier mixture of the granules ranges from 5.5 : 1 to 8.5 : 1.

4. The composition of any preceding claim, wherein
the sugar and/or sugar alcohol is sucrose;
the separating agent is calcium carbonate; and/or
the surfactant is polysorbate, and optionally polysorbate 20.

5. The composition of claim 4, wherein the granules comprise from 30 to 50 wt.-% powdered plant extract;
from 40 to 60 wt.-% sugar and/or sugar alcohol;
from 5 to 10 wt.-% separating agent; and/or
from 0.05 to 1 wt.-% surfactant.

6. The composition of any preceding claim, further comprising mannose, optionally at an amount of 10 to 70 wt.-%.

7. The composition of any preceding claim, further comprising a vitamin, wherein the vitamin is optionally vitamin A and/or vitamin D3.

8. The composition of any preceding claim, further comprising one or more preferably extragranular excipients selected from
edible acids, optionally selected from citric acid, tartaric acid, succinic acid, malic acid or lactic acid, or combinations thereof;
pH-modifying agents, optionally selected from citrates; sweeteners, optionally selected from aspartame, sucralose, cyclamate, neotame, neohesperidin, acesulfame potassium, saccharin, advantame, thaumatin, stevia or stevioside, or mixtures thereof;
natural and/or artificial flavours; and/or anti-foaming agents, optionally selected from medium chain triglycerides (MCTs) with 6 to 12 carbon atoms or simethicone.

9. The composition of any preceding claim, comprising
from 30 to 60 wt.-% mannose;
from 5 to 20 wt.-% powdered birch leaf extract and/or powdered solidago leaf extract;
from 10 to 30 wt.-% sucrose;
from 0.5 to 3 wt.-% calcium carbonate;
from 5 to 25 wt.-% citric acid;
from 3 to 15 wt.-% trimagnesium dicitrate;
from 3 to 15 wt.-% monosodium citrate; and
from 0.001 to 2 wt.-% surfactant.

10. A process for the preparation of the dry flowable composition according to claims 1 to 9, comprising the steps of
(a) mixing the sugar and/or sugar alcohol and the separating agent,
(b) adding a solution comprising an alcohol selected from ethanol or isopropanol, and optionally a surfactant, to the mixture of step a to form a carrier mixture,
(c) adding the powdered plant extract to the carrier mixture of step b, and
(d) drying the product of step c, and
(e) adding to the product of step d mannose and/or vitamin(s) and/or at least one extragranular excipient selected from edible acids, optionally selected from citric acid, tartaric acid, succinic acid, malic acid or lactic acid, or combinations thereof;
pH-modifying agents, optionally selected from citrates;
sweeteners, optionally selected from aspartame, sucralose, cyclamate, neotame, neohesperidin, acesulfame potassium, saccharin, advantame, thaumatin, stevia or stevioside, or mixtures thereof;
natural and/or artificial flavours; and/or
anti-foaming agents, optionally selected from medium chain triglycerides (MCTs) with 6 to 12 carbon atoms or simethicone.

11. A single dose unit or package comprising the dry flowable composition according to claims 1 to 9, wherein
the amount of the total composition is from 2000 to 6000 mg, and
the amount of granules in the composition is from 800 to 1200 mg,
and/or wherein the amount of flavonoids is from 5 to 20 mg.

12. An oral liquid composition obtained from dissolving or dispersing the dry flowable composition according to claims 1 to 9, or the single dose unit or package of claim 11 in water or an aqueous ingestible liquid.

13. The dry flowable composition according to claims 1 to 9, the single dose unit or package of claim 11, or the oral liquid composition of claim 12 for use in the treatment and/or prevention of urinary tract infections.

## Patentansprüche

1. Trockene fließfähige Zusammensetzung, umfassend Granulatpartikel, wobei die Granulatpartikel
(a) einen pulverförmigen Pflanzenextrakt,
(b) eine Trägermischung, umfassend eine wasserlösliche Substanz, die aus einem Zucker und/oder Zuckeralkohol ausgewählt ist, und ein Trennmittel in einem Gewichtsverhältnis von 4:1 bis 11:1, und
(c) ein Tensid;
wobei der Zucker und/oder Zuckeralkohol aus Glucose, Fructose, Mannose, Saccharose, Maltose, Lactose, Maltitol, Sorbitol, Xylitol, Erythritol, Isomalt oder Mischungen davon ausgewählt ist und/oder
das Trennmittel aus Calciumcarbonat, Calciumphosphat, Magnesiumoxid, Magnesiumcarbonat, Fumarsäure oder Mischungen davon ausgewählt ist und wobei die Granulatpartikel
10 bis 70 Gew.-% pulverförmigen Pflanzenextrakt;
20 bis 80 Gew.-% Zucker und/oder Zuckeralkohol;
3 bis 20 Gew.-% Trennmittel und
0,01 bis 3 Gew.-% Tensid
umfassen.

2. Zusammensetzung nach Anspruch 1, wobei es sich bei dem pulverförmigen Pflanzenextrakt um einen Birkenblätter- und/oder Solidagoblätterextrakt handelt, wobei der Birkenblätterextrakt gegebenenfalls mindestens 2 % Flavonoide umfasst.

3. Zusammensetzung nach einem der vorhergehenden Ansprüche, wobei das Gewichtsverhältnis des Zuckers und/oder Zuckeralkohols und des Trennmittels in der Trägermischung der Granulatpartikel im Bereich von 5,5:1 bis 8,5:1 liegt

4. Zusammensetzung nach einem der vorhergehenden Ansprüche, wobei
es sich bei dem Zucker und/oder Zuckeralkohol um Saccharose handelt;
es sich bei dem Trennmittel um Calciumcarbonat handelt und/oder
es sich bei dem Tensid um Polysorbat und gegebenenfalls Polysorbat 20 handelt.

5. Zusammensetzung nach einem der vorhergehenden Ansprüche, wobei die Granulatpartikel
30 bis 50 Gew.-% pulverförmigen Pflanzenextrakt;
40 bis 60 Gew.-% Zucker und/oder Zuckeralkohol;
5 bis 10 Gew.-% Trennmittel und/oder
0,05 bis 1 Gew.-% Tensid
umfassen.

6. Zusammensetzung nach einem der vorhergehenden Ansprüche, ferner umfassend Mannose, gegebenenfalls in einer Menge von 10 bis 70 Gew.-%.

7. Zusammensetzung nach einem der vorhergehenden Ansprüche, ferner umfassend ein Vitamin, wobei es sich bei dem Vitamin gegebenenfalls um Vitamin A und/oder Vitamin D3 handelt.

8. Zusammensetzung nach einem der vorhergehenden Ansprüche, ferner umfassend einen oder mehrere vorzugsweise extragranuläre Hilfsstoffe, die aus
essbaren Säuren, gegebenenfalls ausgewählt aus Citronensäure, Weinsäure, Bernsteinsäure, Äpfelsäure oder Milchsäure oder Kombinationen davon;
Mitteln zur Modifizierung des pH-Werts, gegebenenfalls ausgewählt aus Citraten; Süßungsmitteln, gegebenenfalls ausgewählt aus Aspartam, Sucralose, Cyclamat, Neotam, Neohesperidin, Acesulfam-Kalium, Saccharin, Advantam, Thaumatin, Stevia oder Steviosid oder Mischungen davon;
natürlichen und/oder künstlichen Geschmacksstoffen und/oder
Antischaummitteln, gegebenenfalls ausgewählt aus mittelkettigen Triglyceriden (Medium Chain Triglycerides, MCT) mit 6 bis 12 Kohlenstoffatomen oder Simethicon, ausgewählt sind.

9. Zusammensetzung nach einem der vorhergehenden Ansprüche, umfassend 30 bis 60 Gew.-% Mannose;
5 bis 20 Gew.-% pulverförmigen Birkenblätterextrakt und/oder Solidagoblätterextrakt;
10 bis 30 Gew.-% Saccharose;
0,5 bis 3 Gew.-% Calciumcarbonat;
5 bis 25 Gew.-% Citronensäure;
3 bis 15 Gew.-% Trimagnesiumdicitrat;
3 bis 15 Gew.-% Mononatriumcitrat und
0,001 bis 2 Gew.-% Tensid.

10. Verfahren zur Herstellung der trockenen fließfähigen Zusammensetzung gemäß den Ansprüchen 1 bis 9, das folgende Schritte umfasst:
(a) Mischen des Zuckers und/oder Zuckeralkohols und des Trennmittels,
(b) Hinzufügen einer Lösung, die einen aus Ethanol oder Isopropanol ausgewählten Alkohol und gegebenenfalls ein Tensid umfasst, zu der Mischung aus Schritt a zur Bildung einer Trägermischung,
(c) Hinzufügen des pulverförmigen Pflanzenextrakts zu der Trägermischung aus Schritt b und
(d) Trocknen des Produkts aus Schritt c und
(e) Hinzufügen von Mannose und/oder Vitamin(en) und/oder mindestens eines extragranulären Hilfsstoffs, der aus
essbaren Säuren, gegebenenfalls ausgewählt aus Citronensäure, Weinsäure, Bernsteinsäure, Äpfelsäure oder Milchsäure oder Kombinationen davon;
Mitteln zur Modifizierung des pH-Werts, gegebenenfalls ausgewählt aus Citraten; Süßungsmitteln, gegebenenfalls ausgewählt aus Aspartam, Sucralose, Cyclamat, Neotam, Neohesperidin, Acesulfam-Kalium, Saccharin, Advantam, Thaumatin, Stevia oder Steviosid oder Mischungen davon;
natürlichen und/oder künstlichen Geschmacksstoffen und/oder
Antischaummitteln, gegebenenfalls ausgewählt aus mittelkettigen Triglyceriden (Medium Chain Triglycerides, MCT) mit 6 bis 12 Kohlenstoffatomen oder Simethicon,
ausgewählt ist,
zu dem Produkt aus Schritt d.

11. Einzeldosiseinheit oder -packung, umfassend die trockene fließfähige Zusammensetzung gemäß den Ansprüchen 1 bis 9, wobei
die Menge der gesamten Zusammensetzung etwa 2000 bis etwa 6000 mg beträgt und
die Menge an Granulatpartikeln in der Zusammensetzung 800 bis 1200 mg beträgt und/oder wobei die Menge an Flavonoiden 5 bis 20 mg beträgt

12. Orale flüssige Zusammensetzung, erhalten durch Lösen oder Dispergieren der trockenen fließfähigen Zusammensetzung gemäß den Ansprüchen 1 bis 9 oder der Einzeldosiseinheit oder -packung nach Anspruch 11 in Wasser oder einer wässrigen einnehmbaren Flüssigkeit

13. Trockene fließfähige Zusammensetzung gemäß den Ansprüchen 1 bis 9, Einzeldosiseinheit oder -packung nach Anspruch 11 oder orale flüssige Zusammensetzung nach Anspruch 12 zur Verwendung bei der Behandlung und/oder Prävention von Harnwegsinfektionen.

## Revendications

1. Composition sèche apte à l'écoulement qui comprend des granulés, lesdits granulés comprenant
(a) un extrait végétal en poudre,
(b) un mélange porteur comprenant une substance hydrosoluble choisie parmi un sucre et/ou un alcool de sucre et un agent de séparation dans un rapport en poids de 4:1 à 11:1, et
(c) un tensioactif ;
le sucre et/ou l'alcool de sucre étant choisi parmi le glucose, le fructose, le mannose, le saccharose, le maltose, le lactose, le maltitol, le mannitol, le sorbitol, le xylitol, l'érythritol, l'isomalt ou des mélanges de ceux-ci ; et/ou
l'agent de séparation étant choisi parmi le carbonate de calcium, le phosphate de calcium, l'oxyde de magnésium, le carbonate de magnésium, l'acide fumarique ou des mélanges de ceux-ci, et les granulés comprenant
de 10 à 70 % en poids d'extrait végétal en poudre ;
de 20 à 80 % en poids de sucre et/ou d'alcool de sucre ;
de 3 à 20 % en poids d'agent de séparation ; et
de 0,01 à 3 % en poids d'agent tensioactif.

2. Composition selon la revendication 1, l'extrait de plante en poudre étant une feuille de bouleau et/ou un extrait de feuille de solidago, l'extrait de feuille de bouleau comprenant éventuellement au moins 2 % de flavonoïdes.

3. Composition selon l'une quelconque des revendications précédentes, le rapport en poids du sucre et/ou de l'alcool de sucre et de l'agent de séparation dans le mélange porteur des granulés étant compris entre 5,5:1 et 8,5:1.

4. Composition selon l'une quelconque des revendications précédentes,
le sucre et/ou l'alcool de sucre étant le saccharose ;
l'agent de séparation étant le carbonate de calcium ; et/ou
le tensioactif étant le polysorbate, et éventuellement le polysorbate 20.

5. Composition selon la revendication 4, les granulés comprenant
de 30 à 50 % en poids d'extrait végétal en poudre ;
de 40 à 60 % en poids de sucre et/ou d'alcool de sucre ;
de 5 à 10 % en poids d'agent de séparation ; et/ou
de 0,05 à 1 % en poids d'agent tensioactif.

6. Composition selon l'une quelconque des revendications précédentes, comprenant en outre du mannose, éventuellement dans une quantité de 10 à 70 % en poids.

7. Composition selon l'une quelconque des revendications précédentes, comprenant en outre une vitamine, la vitamine étant éventuellement la vitamine A et/ou la vitamine D3.

8. Composition selon l'une quelconque des revendications précédentes, comprenant en outre un ou plusieurs excipients préférentiellement extragranulaires choisis parmi
les acides comestibles, éventuellement choisis parmi l'acide citrique, l'acide tartrique, l'acide succinique, l'acide malique ou l'acide lactique, ou des combinaisons de ceux-ci ;
des agents modificateurs de pH, éventuellement choisis parmi les citrates ;
des édulcorants, éventuellement choisis parmi l'aspartame, le sucralose, le cyclamate, le néotame, la néohespéridine, l'acésulfame potassique, la saccharine, l'advantame, la thaumatine, la stévia ou le stévioside, ou des mélanges de ceux-ci ;
des arômes naturels et/ou artificiels ; et/ou
des agents anti-moussants, éventuellement choisis parmi les triglycérides à chaîne moyenne (MCT) de 6 à 12 atomes de carbone ou la siméthicone.

9. Composition selon l'une quelconque des revendications précédentes, comprenant de 30 à 60 % en poids de mannose ;
de 5 à 20 % en poids d'extrait de feuille de bouleau en poudre et/ou d'extrait de feuille de solidago en poudre ;
de 10 à 30 % en poids de saccharose ;
de 0,5 à 3 % en poids de carbonate de calcium ;
de 5 à 25 % en poids d'acide citrique ;
de 3 à 15 % en poids de dicitrate de trimagnésium ;
de 3 à 15 % en poids de citrate monosodique ; et
de 0,001 à 2 % en poids d'agent tensioactif.

10. Procédé de préparation de la composition sèche apte à l'écoulement selon les revendications 1 à 9, le procédé comprenant les étapes suivantes
(a) mélanger le sucre et/ou l'alcool de sucre et l'agent de séparation,
(b) ajouter une solution comprenant un alcool choisi parmi l'éthanol ou l'isopropanol, et éventuellement un tensioactif, au mélange de l'étape a pour former un mélange porteur,
(c) ajouter l'extrait végétal en poudre au mélange porteur de l'étape b, et
(d) sécher le produit de l'étape c, et
(e) ajouter au produit de l'étape d du mannose et/ou une ou des vitamines et/ou au moins un excipient extragranulaire choisi parmi les acides comestibles, éventuellement choisis parmi l'acide citrique, l'acide tartrique, l'acide succinique, l'acide malique ou l'acide lactique, ou des combinaisons de ceux-ci ;
des agents modificateurs de pH, éventuellement choisis parmi les citrates ;
les édulcorants, éventuellement choisis parmi l'aspartame, le sucralose, le cyclamate, le néotame, la néohespéridine, l'acésulfame potassique, la saccharine, l'advantame, la thaumatine, la stévia ou le stévioside, ou des mélanges de ceux-ci ; arômes naturels et/ou artificiels ; et/ou
des agents anti-moussants, éventuellement choisis parmi les triglycérides à chaîne moyenne (MCT) de 6 à 12 atomes de carbone ou la siméthicone.

11. Unité ou emballage unidose comprenant la composition sèche apte à l'écoulement selon les revendications 1 à 9,
la quantité de la composition totale étant de 2000 à 6000 mg, et
la quantité de granulés dans la composition étant de 800 à 1200 mg,
et/ou la quantité de flavonoïdes étant de 5 à 20 mg.

12. Composition liquide orale obtenue par dissolution ou dispersion de la composition sèche apte à l'écoulement selon les revendications 1 à 9, ou l'unité ou l'emballage unidose selon la revendication 11 dans de l'eau ou un liquide aqueux ingérable.

13. Composition sèche apte à l'écoulement selon les revendications 1 à 9, unité ou l'emballage unidose selon la revendication 11, ou composition liquide orale selon la revendication 12 destinées à être utilisée dans le traitement et/ou la prévention d'infections des voies urinaires.
